# EUROPEAN PATENT APPLICATION

(11) **EP 3 338 639 A1**
(43) Date of publication of application: **27.06.2018**
(21) Application number: 17206591.4
(22) Date of filing: 12.12.2017
(51) Int. Cl.: A61B 8/00

(54) **ULTRASONIC PROBE**

(30) Priority: 14.12.2016 KR 20160170194
(71) Applicant: Samsung Medison Co., Ltd., Hongcheon-gun, Gangwon-do, 25108 (KR)
(72) Inventor: CHOI, Min Seog, Seoul (KR); SONG, Jong Keun, Gyeonggi-do (KR)
(74) Representative: Grootscholten, Johannes A.M.

(57) **Abstract**

Disclosed herein is a ultrasonic probe (100) that includes a case (101), a transducer array (104), a driving device (105) disposed in the case and configured to drive the transducer array, a thermal conduction member (110) connected to the driving device and having thermal conductivity, a thermoelectric element (120) comprising a heat absorbing part (121) and a heat dissipating part (122), the heat absorbing part disposed to face the thermal conduction member to cool the thermal conduction member, a fluid cooling apparatus (130) configured to cool the heat dissipating part of the thermoelectric element by circulating a fluid of at least one portion of the inside of the case to the outside of the case, and a partition (140) configured to divide an inner space of the case into a first space in which the heat absorbing part of the thermoelectric element is disposed and a second space in which the heat dissipating part is disposed.

## Description

### BACKGROUND

### 1. Field

Embodiments of the present disclosure relate to an ultrasonic probe, and more particularly, to an ultrasonic probe having an improved heat dissipation structure.

### 2. Description of the Related Art

An ultrasound imaging apparatus radiates ultrasonic signals toward a target region inside a body of an object from a surface of the object and then collects reflected ultrasonic signals (ultrasonic echo signals) to non-invasively acquire tomograms of soft tissues or images of blood streams using information thereon.

The ultrasound imaging apparatus is relatively small in size, inexpensive, displays an image in real time, and has high stability due to no radiation exposure as compared with other diagnostic imaging apparatuses such as X-ray diagnosis apparatuses, computerized tomography (CT) scanners, magnetic resonance imaging (MRI) apparatuses, and nuclear medicine diagnosis apparatuses. Thus, the ultrasound imaging apparatus has been widely used for heart diagnosis, celiac diagnosis, urinary diagnosis, and obstetric diagnosis.

The ultrasound imaging apparatus may include an ultrasonic probe to transmit and receive ultrasound. The ultrasonic probe may transmit ultrasound to an object via a transducer array and receive echo ultrasound reflected by the object.

When current is supplied to a transducer, the transducer oscillates and produces ultrasound. In this case, heat is generated by oscillation of the transducer. In particular, when a transducer array includes a plurality of transducer elements, an amount of heat generated thereby may increase geometrically. Thus, the ultrasonic probe may include a heat dissipating device to efficiently dissipate heat.

### SUMMARY

Therefore, it is an aspect of the present disclosure to provide an ultrasonic probe having improved heat dissipating effects.

Additional aspects of the disclosure will be set forth in part in the description which follows and, in part, will be obvious from the description, or may be learned by practice of the disclosure.

In accordance with one aspect of the present disclosure, an ultrasonic probe includes a case, a transducer array disposed in the case and configured to generate ultrasound, a driving device disposed in the case and configured to drive the transducer array, a thermal conduction member connected to the driving device and having thermal conductivity, a thermoelectric element comprising a heat absorbing part and a heat dissipating part, the heat absorbing part disposed to face the thermal conduction member to cool the thermal conduction member, a fluid cooling apparatus configured to cool the heat dissipating part of the thermoelectric element by circulating a fluid of at least one portion of the inside of the case to the outside of the case, and a partition configured to divide an inner space of the case into a first space in which the heat absorbing part of the thermoelectric element is disposed and a second space in which the heat dissipating part is disposed.

The partition may extend from an inner surface of the case toward the thermoelectric element to be in contact with the thermoelectric element.

The partition may be in contact with a boundary of the heat absorbing part and the heat dissipating part of the thermoelectric element.

The fluid cooling apparatus may be provided to adjust an amount of the fluid circulating to the outside of the case in proportion to an amount of heat generated by the driving device.

The thermoelectric element may adjust a temperature difference between the heat absorbing part and the heat dissipating part in proportion to an amount of heat generated by the driving device.

The fluid cooling apparatus may include an inlet pipe configured to move a fluid outside the case into the second space of the case, and an introduction device connected to the inlet pipe and configured to forcibly move the fluid outside the case into the second space of the case.

The ultrasonic probe may further include a cable penetrating one side of the case to supply power to the driving device, wherein the inlet pipe may be disposed inside the cable.

The fluid cooling apparatus may include an outlet pipe configured to discharge the fluid of the second space of the case out of the case.

The fluid cooling apparatus may include a discharge device connected to the outlet pipe and configured to forcibly move the fluid of the second space of the case out of the case.

The fluid cooling apparatus may include a discharge hole formed at one portion of the case constituting the second space of the case and penetrating the case to discharge the fluid of the second space of the case out of the case.

The ultrasonic probe may further include a cooling fin disposed adjacent to the heat dissipating part of the thermoelectric element.

The ultrasonic probe may further include a printed circuit board (PCB) electrically connected to the driving device, wherein the PCB may be disposed in the first space of the case.

The ultrasonic probe may further include a thermal conduction block having a size corresponding to the driving device in contact with one surface of the driving device facing the thermal conduction member.

The transducer array may include transducer elements arranged two-dimensionally.

The thermal conduction member may include a heat pipe.

In accordance with another aspect of the present disclosure, an ultrasonic probe includes a case, a transducer array disposed in the case and configured to generate ultrasound, a driving device configured to drive the transducer array and generate heat when driving the transducer array, a thermal conduction member configured to conduct heat generated by the driving device from the transducer array, a thermoelectric element comprising a heat absorbing part in contact with the thermal conduction member and a heat dissipating part disposed at an opposite side to the heat absorbing part, a fluid cooling apparatus configured to forcibly supply a fluid to the heat dissipating part to cool the heat dissipating part of the thermoelectric element, and a partition configured to extend to the thermoelectric element from an inner surface of the case, wherein the fluid cooling apparatus adjusts an amount of the supplied fluid in proportion to an amount of heat generated by the driving device.

The fluid cooling apparatus may include an outlet pipe configured to discharge the fluid supplied to the heat dissipating part out of the case.

The fluid cooling apparatus may include a discharge hole penetrating the case to discharge the fluid supplied to the heat dissipating part out of the case.

The ultrasonic probe may further include a cooling fin disposed to be in contact with at least one portion of the heat dissipating part of the thermoelectric element.

In accordance with still another aspect of the present disclosure, an ultrasonic probe includes a case, a transducer array disposed in the case and configured to generate ultrasound, a driving device disposed in the case and configured to drive the transducer array, a thermal conduction member connected to the driving device and having thermal conductivity, a thermoelectric element comprising a heat absorbing part and a heat dissipating part, the heat absorbing part disposed to face the thermal conduction member to cool the thermal conduction member, and a fluid cooling apparatus configured to cool the heat dissipating part of the thermoelectric element by circulating air of at least one portion in the case to the outside of the case, wherein the thermoelectric element divides an inner space of the case into a first space in which the heat absorbing part is disposed and a second space in which the heat dissipating part is disposed.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a perspective view illustrating an ultrasound imaging apparatus according to an embodiment of the present disclosure.
FIG. 2 is a view illustrating the ultrasonic probe of FIG. 1.
FIG. 3 is a cross-sectional view of the ultrasonic probe of FIG. 2.
FIG. 4 is a block diagram for describing a method of controlling a thermoelectric element and a fluid cooling apparatus of the ultrasonic probe illustrated in FIG. 3.
FIG. 5 is a cross-sectional view of an ultrasonic probe 200 according to another embodiment of the present disclosure.
FIG. 6 is a cross-sectional view of an ultrasonic probe 300 according to another embodiment of the present disclosure.
FIG. 7 is a cross-sectional view of an ultrasonic probe 400 according to another embodiment of the present disclosure.
FIG. 8 is a cross-sectional view of an ultrasonic probe 500 according to another embodiment of the present disclosure.
FIG. 9 is a cross-sectional view of an ultrasonic probe 600 according to another embodiment of the present disclosure.
FIG. 10 is a cross-sectional view of an ultrasonic probe 700 according to another embodiment of the present disclosure.
FIG. 11 is a cross-sectional view of an ultrasonic probe 800 according to another embodiment of the present disclosure.

### DETAILED DESCRIPTION

Reference will now be made in detail to the embodiments of the present disclosure, examples of which are illustrated in the accompanying drawings, wherein like reference numerals refer to like elements throughout.

Also, like reference numerals or symbols provided in the drawings of the present specification represent members or components that perform substantially the same functions.

Throughout the specification, the terms used are merely used to describe particular embodiments, and are not intended to limit the present disclosure. As used herein, the singular forms "a", "an" and "the" are intended to include the plural forms as well, unless otherwise stated. Also, it is to be understood that the terms such as "include" and "have" are intended to indicate the existence of the features, numbers, operations, components, parts, or combinations thereof disclosed in the specification, and are not intended to preclude the possibility that one or more other features, numbers, operations, components, parts, or combinations thereof may exist or may be added.

It will be understood that, although the terms "first", "second", etc., may be used herein to describe various elements, these elements should not be limited by these terms. The above terms are used only to distinguish one component from another. For example, a first component discussed below could be termed a second component, and similarly, the second component may be termed the first component without departing from the teachings of this disclosure. As used herein, the term "and/or" includes any and all combinations of one or more of the associated listed items.

Meanwhile, the terms used throughout the specification "front", "rear", "upper", "lower", and the like are defined based on the drawings and the shape and position of each element are not limited by these terms.

Hereinafter, embodiments of the present disclosure will be described in detail with reference to the accompanying drawings.

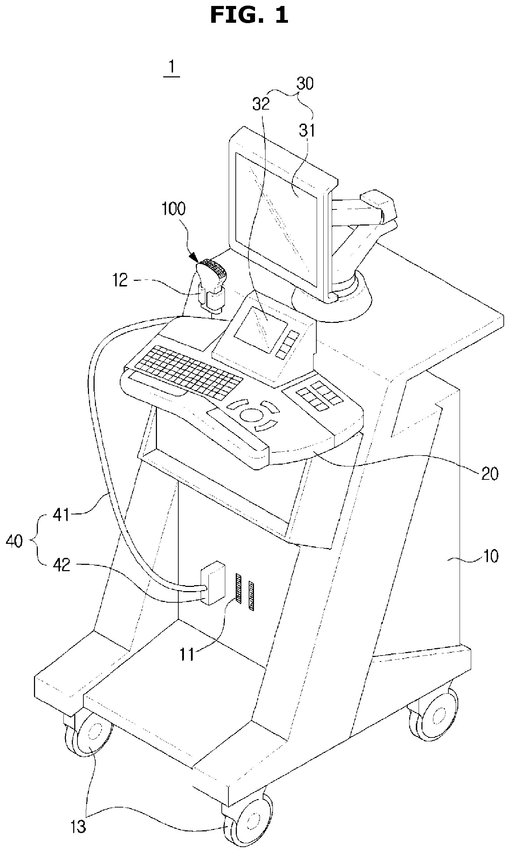

FIG. 1 is a perspective view illustrating an ultrasound imaging apparatus 1 according to an embodiment of the present disclosure.

Referring to FIG. 1, the ultrasound imaging apparatus 1 may include a main body 10, an input device 20, a display 30, and an ultrasonic probe 100.

At least one connection unit 11 may be provided at one side of the main body 10. A connector 42 connected to a cable 41 may be physically coupled to the connection unit 11.

The main body 10 may have a holder 12 in which the ultrasonic probe 100 is placed. An examiner may store the ultrasonic probe 100 to be held by the holder 12 when the ultrasound imaging apparatus 1 is not in use.

The main body 10 may include a moving device 13 provided at a lower part thereof to move the ultrasound imaging apparatus 1. The moving device 13 may be a plurality of casters provided at the bottom of the main body 10. The casters may be aligned to allow the main body 10 to move in a given direction or may be freely movably provided to allow the ultrasound imaging apparatus 1 to move in any directions. The moving device 13 may include a locking device (not shown) to stop the ultrasound imaging apparatus 1 at a given position. Such an ultrasound imaging apparatus 1 is referred to as a cart type ultrasound imaging apparatus.

However, the ultrasound imaging apparatus may also be a portable ultrasound imaging apparatus that can be carried during a long distance movement which is different from that illustrated in FIG. 1. Here, the portable ultrasound imaging apparatus may not be provided with a moving device. Examples of the portable ultrasound imaging apparatus may include a PACS Viewer, a smart phone, a laptop computer, a PDA, a tablet PC, and the like, without being limited thereto.

The ultrasonic probe 100 may transmit and receive ultrasonic signals in a contact state with the surface of an object. Particularly, the ultrasonic probe 100 may transmit an ultrasonic signal to a predetermined region inside the object in response to a transmit signal received from the main body 10, receive an ultrasonic echo signal reflected by the predetermined region of the object, and transmit the received signal to the main body upon receiving the ultrasonic echo signal. In this regard, the ultrasonic echo signal may be a radio frequency (RF) signal reflected by the object. However, the ultrasonic echo signal is not limited thereto and may include all of the reflected ultrasonic signals reflected by the object.

Meanwhile, the object may be a human or animal without being limited thereto and may also be any object whose internal structure may be imaged by using the ultrasonic signals.

The ultrasonic probe 100 may be connected to the main body 10 via a connecting member 40. The connecting member 40 may include the cable 41 and the connector 42. The ultrasonic probe 100 may be provided at one side of the cable 41 and the connector 42 may be provided at the other side of the cable 41. The connector 42 may be detachably coupled to the connection unit 11 provided in the main body 10. Accordingly, the ultrasonic probe 100 may be connected to the main body 10.

The ultrasonic probe 100 may be connected to the main body 10 via the connecting member 40 to receive various signals required to control the ultrasonic probe 100 in a wired communication network therefrom or transmit analog signals or digital signals corresponding to the ultrasonic echo signals received by the ultrasonic probe 100 thereto.

Specifically, the wired communication network refers to a communication network capable of transmitting and receiving signals via a cable. According to an embodiment, the main body 10 may exchange various signals with the ultrasonic probe 100 via a wired communication network such as Peripheral Component Interconnect (PCI), PCI-express, and Universe Serial Bus (USB), without being limited thereto.

However, the present embodiment is not limited thereto and the ultrasonic probe 100 may also be connected to the main body 10 via a wireless communication network to receive various signals required to control the ultrasonic probe 100 therefrom or transmit analog signals or digital signals corresponding to the ultrasonic echo signals received by the ultrasonic probe 100 thereto. The wireless communication network refers to any type of network that establishes connections without cables. In this case, the main body 10 may perform wireless communications with the ultrasonic probe 100 via at least one of a short-range wireless communication module and a mobile communication module.

The short-range communication module refers to a module for short-range communication within a predetermined distance. For example, short-range communication technologies may include Wireless LAN, Wi-Fi, Bluetooth, Zigbee, Wi-Fi- Direct (WFD), Infrared Data Association (IrDA), Bluetooth Low Energy (BLE), Near Field Communication (NFC), and the like, without being limited thereto.

The mobile communication module may exchange wireless signals with at least one of a base station, an external terminal, and a server on a mobile communication network. In this regard, the wireless signals refer to signals including various types of data. That is, the main body 10 may exchange signals including various types of data with the ultrasonic probe 100 via at least one of the base station and the server.

For example, the main body 10 may exchange signals including various types of data with the ultrasonic probe 100 via a base station on a mobile communication network such as 3G and 4G telecommunication networks. In addition, the main body 10 may exchange data with a hospital server or other medical devices in a hospital connected via a Picture Archiving and Communication System (PACS). In addition, the main body 10 may transmit and receive data in accordance with Digital Imaging and Communication in Medicine (DICOM) standards, without being limited thereto.

The configuration of the ultrasonic probe 100 will be described in more detail later.

Meanwhile, the main body 10 may be provided with an image processor configured to convert echo ultrasound received by the ultrasonic probe into an ultrasound image. The image processor may be implemented as a hardware processor such as a microprocessor or a software processor executed on a hardware platform.

The image processor may generate an ultrasound image through scan conversion of echo ultrasound. In this regard, the ultrasound image may include not only a gray scale image acquired by scanning the object in an amplitude mode (A-mode), a brightness mode (B-mode), and a motion mode (M-mode) but also a Doppler image representing an image of a moving object by using the Doppler Effect. The Doppler image may include a blood stream Doppler image showing a flow of blood (color Doppler image), a tissue Doppler image showing movement of tissues, and a spectral Doppler image illustrating a speed of a moving object as waveforms.

The image processor may extract a B-mode component from the echo ultrasound received by the ultrasonic probe to generate a B-mode image. The image processor may generate an ultrasound image expressed in such a manner that the intensity of the echo ultrasound is warped based on the B-mode component.

Similarly, the image processor may extract a Doppler component from the echo ultrasound and generate a Doppler image that expresses the motion of the object in a color or waveform based on the extracted Doppler component.

Furthermore, the image processor may generate a 3D ultrasound image by volume rendering volume data acquired from echo ultrasound or an elastic image in which the degree of deformation of the object according to pressure is imaged. In addition, the image processor may display various additional information on the ultrasound image in texts or graphics.

Meanwhile, the generated ultrasound image may be stored in a memory inside or outside the main body. Alternatively, the ultrasound image may be stored in a web storage or a cloud server which performs a storage function on the web.

The main body 10 may include an input device 20. The input device 20 may be provided in the form of keyboard, foot switch, foot pedal, or the like. When the input device 20 is a keyboard, the keyboard may be provided at the upper portion of the main body 10. The keyboard may include at least one of a switch, a key, a joystick, and a trackball. When the input device 20 is a foot switch or a foot pedal, the foot switch or the foot pedal may be provided at a lower portion of the main body 10.

Alternatively, the input device 20 may also be implemented using a software component such as a graphic user interface. In this case, the input device 20 may be displayed via the display 30 and the display 30 may be implemented using a touch screen type.

The examiner may control the operation of the ultrasound imaging apparatus 1 by using the input device 20. For example, a mode selection command to select A-mode, B-mode, M-mode, or Doppler image may be input thereby. Furthermore, an ultrasound diagnosis start command may be input thereby. A command input via the input device 20 may be transmitted to the main body 10 via a wired or wireless communication network.

The display 30 may include a first display 31 and a second display 32. The display 30 may display an ultrasound image acquired during ultrasound diagnosis. In addition, the display 30 may display applications related to the operation of the ultrasound imaging apparatus 1. For example, the first display 31 may display an ultrasound image acquired during ultrasound diagnosis. The second display 32 may display items related to the operation of the ultrasound imaging apparatus.

The first display 31 and/or the second display 32 may be implemented in various ways using a cathode ray tube (CRT), a liquid crystal display (LCD), a light emitting diode (LED), a plasma display panel (PDP), an organic light emitting diode (OLED), and the like. The first display 31 and/or the second display 32 may be provided in a state of being coupled to or separated from the main body 10.

Although FIG. 1 illustrates that the display 30 includes the first display 31 and the second display 32, the first display 31 or the second display 32 may be omitted if required.

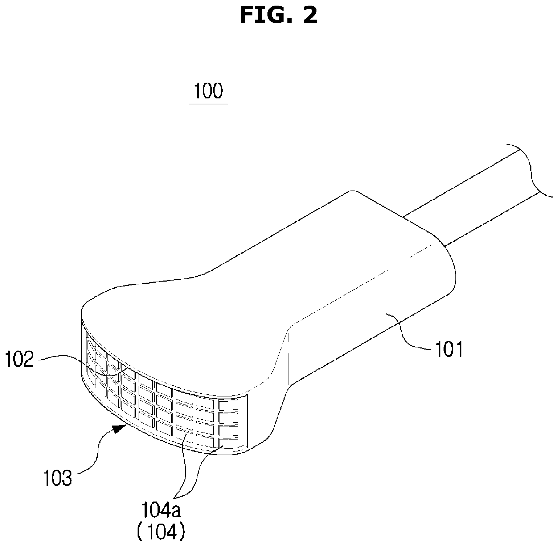

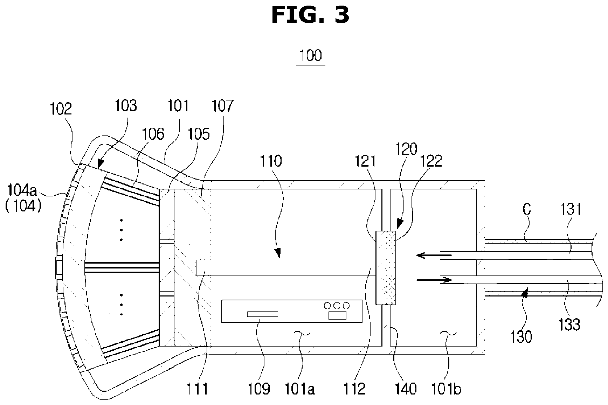

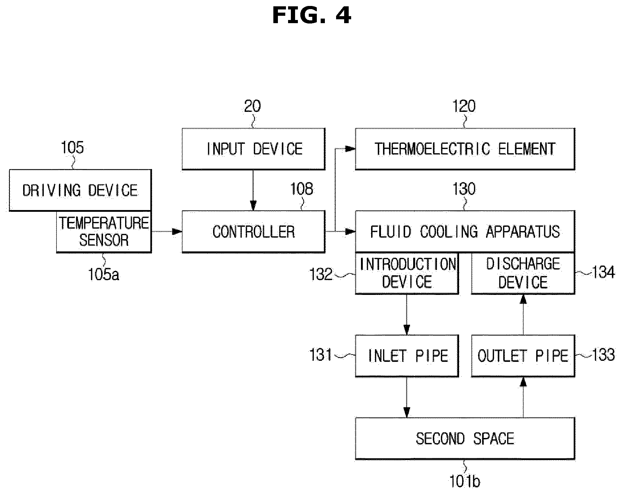

FIG. 2 is a view illustrating the ultrasonic probe 100 of FIG. 1. FIG. 3 is a cross-sectional view of the ultrasonic probe 100 of FIG. 2. FIG. 4 is a block diagram for describing a method of controlling a thermoelectric element 120 and a fluid cooling apparatus 130 of the ultrasonic probe 100 illustrated in FIG. 3. In this regard, FIG. 3 is a cross-sectional view of the ultrasonic probe 100 of FIG. 2 taken along a substantially horizontal line.

Referring to FIGS. 2 to 4, the ultrasonic probe 100 may include a case 101, an acoustic module 103 disposed at a portion in the case 101, and a driving device 105 configured to drive the acoustic module 103.

A part of the acoustic module 103 may be exposed to the outside of the case 101 via an opening 102 formed at a front portion of the case 101. Accordingly, the ultrasonic probe 100 may transmit and receive ultrasonic signals in a contact state with the surface of the object.

The driving device 105 may be connected to the acoustic module 103 via an interposer 106 to drive the acoustic module 103. As the driving device 105 drives the acoustic module 103, a transducer array 104 may be driven.

The acoustic module 103 may include the transducer array 104 that converts electrical signals into ultrasonic signals and vice versa to transmit ultrasonic signals toward the inside of the object. The transducer array 104 may include a plurality of transducer elements 104a.

The ultrasonic probe 100 generates ultrasonic signals by using the transducer array 104, transmits the ultrasonic signals toward a target region inside the object, and receives ultrasonic echo signals reflected by the target region inside the object by using the transducer array 104.

When the ultrasonic echo signal arrives at the transducer array 104, the transducer array 104 oscillates at a predetermined frequency corresponding to a frequency of the ultrasonic echo signal to output AC voltage having a frequency corresponding to the oscillation frequency of the transducer array 104. Thus, the transducer array 104 may convert the received ultrasonic echo signal into an echo signal that is an electrical signal.

Each of the transducer elements 14a constituting the transducer array 104 may convert ultrasonic signals into electrical signals and vice versa. To this end, the transducer elements 104a may be implemented using a magnetostrictive ultrasonic transducer that uses a magnetostrictive effect of a magnetic material, a piezoelectric ultrasonic transducer or a piezoelectric micromachined ultrasonic transducer (pMUT) which uses a piezoelectric effect of a material, or a capacitive micromachined ultrasonic transducer (cMUT) that transmits and receives ultrasound by using oscillation of hundreds of or thousands of micromachined thin films.

Meanwhile, the transducer elements 104a of the ultrasonic probe 100 may be arranged in a convex shape as illustrated in FIG. 2 or may be linearly arranged although not shown therein. In both cases, the basic operation principle of the ultrasonic probe 100 is the same. However, since the ultrasonic signals are emitted from the transducer elements 104a in a fan shape in the case of the ultrasonic probe 100 in which the transducer elements 104a are arranged in the convex shape, an ultrasound image may also be generated in a fan shape.

Meanwhile, as illustrated in FIG. 2, the transducer array 104 may be provided as a two-dimensional (2D) transducer array in which the transducer elements 104a are two-dimensionally arranged. When the transducer array 104 is provided as the 2D transducer array, the ultrasonic probe 100 may acquire a three-dimensional (3D) image of the inside of the object. On the contrary, although not shown in the drawings, the transducer array 104 may be provided as a one-dimensional (1D) transducer array. When the transducer array 104 is provided as a 1D transducer array, the ultrasonic probe 100 may acquire volume information of the inside of the object while mechanically moving the 1D transducer array and transmit ultrasonic echo signals capable of generating a 3D ultrasound image to the main body 10.

Meanwhile, in the ultrasonic probe 100, when the driving device 105 drives the acoustic module 103 to allow the transducer elements 104a to transmit or receive ultrasound, the driving device 105 and/or the transducer elements 104a may oscillate. This oscillation generates heat. Particularly, when the transducer array 104 is a 2D transducer array, an amount of heat generated by the transducer array 104 and the driving device 105 that drives the transducer array 104 increases. The heat may cause inconvenience to a patient when delivered to the patient through the transducer array 104 or cause adverse effects on a printed circuit board (PCB) 109 to deteriorate the quality of image when remaining in the main body 10.

Thus, the ultrasonic probe 100 according to the present embodiment may include a thermal conduction member 110 connected to the driving device 105, a thermoelectric element 120 configured to cool the thermal conduction member 110, a fluid cooling apparatus 130 configured to cool the thermoelectric element 120, and a partition 140 configured to divide an inner space of the case 101.

The thermal conduction member 110 may be configured to include a material having a high thermal conductivity. The thermal conduction member 110 may be connected to the driving device 105 to allow heat to be transferred from the driving device 105. The thermal conduction member 110 may conduct heat generated in the driving device 105 backward from the front of the ultrasonic probe 100.

Referring to FIG. 3, one end 111 of the thermal conduction member 110 may be connected to the driving device 105 via a thermal conduction block 107. Particularly, the one end 111 of the thermal conduction member 110 may be inserted into one surface of the thermal conduction block 107, and the other surface of the thermal conduction block 107 opposite to the one surface may be brought into contact with the driving device 105. Accordingly, heat generated in the driving device 105 may be transferred to the thermal conduction member 110 via the thermal conduction block 107.

The thermal conduction block 107 may be configured to include a high thermal conductivity. The thermal conduction block 107 may be provided such that the other surface thereof in contact with the driving device 105 has a size substantially the same as that of a contact surface of the driving device 105. That is, the thermal conduction block 107 having the size corresponding to one surface of the driving device 105 facing the thermal conduction member 110 may be brought into contact with the driving device 105. Meanwhile, the thermal conduction block 107 may be omitted, if required.

The other end 112 of the thermal conduction member 110 opposite to the one end 111 may be connected to the thermoelectric element 120. The other end 112 of the thermal conduction member 110 may contact a heat absorbing part 121 of the thermoelectric element 120.

According to this configuration, the one end 111 of the thermal conduction member 110 is connected to the driving device 105 having a relatively high temperature and the other end 112 is connected to the heat absorbing part 121 of the thermoelectric element 120 having a relatively low temperature so that heat is conducted from the left to the right in the drawing. That is, the thermal conduction member 110 may conduct heat generated in the driving device 105 to the heat absorbing part 121 of the thermoelectric element 120.

The thermal conduction member 110 may be provided in the form of a rod or a pipe. The thermal conduction member 110 may be provided as a heat pipe.

The thermoelectric element 120 may include the heat absorbing part 121 disposed at one side and a heat dissipating part 122 disposed at the other side opposite to the heat absorbing part 121. The thermoelectric element 120 is a device using a Peltier effect in which the heat absorbing part 121 and the heat dissipating part 122 are formed by supplying a current. The thermoelectric element 120 is well known in the art, and thus detailed descriptions thereof will be omitted.

The thermoelectric element 120 may cool the other end 112 of the thermal conduction member 110. The heat absorbing part 121 of the thermoelectric element 120 may be disposed to face the thermal conduction member 110. Particularly, as the heat absorbing part 121 of the thermoelectric element 120 is in contact with the thermal conduction member 110, the other end 112 of the thermal conduction member 110 may be cooled.

The heat dissipating part 122 of the thermoelectric element 120 may be cooled by the fluid cooling apparatus 130 which will be described later.

Referring to FIG. 4, the thermoelectric element 120 may be provided to control a temperature difference between the heat absorbing part 121 and the heat dissipating part 122 in proportion to the amount of heat generated by the driving device 105. That is, when a temperature sensor 105a measures a temperature of the driving device 105 and transmits the measured temperature of the driving device 105 to a controller 108, the controller 108 may adjust the intensity of current supplied to the thermoelectric element 120. Specifically, when the temperature of the driving device 105 is relatively high due to a large amount of heat generated by the driving device 105, the controller 108 may increase the intensity of current supplied to the thermoelectric element 120 to lower the temperature of the heat absorbing part 121 relatively more. On the contrary, when the temperature of the driving device 105 is relatively low due to a small amount of heat generated by the driving device 105, the controller 108 may decrease the intensity of current supplied to the thermoelectric element 120 to lower the temperature of the heat absorbing part 121 relatively less.

According to this configuration, the ultrasonic probe 100 according to the present embodiment may quickly conduct heat generated in the driving device 105 in a direction away from the driving device 105.

The fluid cooling apparatus 130 may cool the heat dissipating part 122 of the thermoelectric element 120 by circulating a fluid from at least one part of the case 101 out of the case 101. Particularly, the fluid cooling apparatus 130 may supply a fluid to the heat dissipating part 122 of the thermoelectric element 120 from the outside. The fluid cooling apparatus 130 may supply the fluid into a second space 101b in the case 101. In this regard, the fluid may be air. The fluid cooling apparatus 130 according to the present embodiment may include an inlet pipe 131, an introduction device 132, an outlet pipe 133, and a discharge device 134.

The inlet pipe 131 may be provided to move the fluid into the second space 101b of the case 101 from the outside of the case 101. That is, the inlet pipe 131 may guide the fluid outside the case 101 into the case 101. The inlet pipe 131 may be disposed inside a cable C that penetrates one side of the case 101 to supply power to the driving device 105. Accordingly, the inlet pipe 131 may penetrate the case 101. The inlet pipe 131 may be disposed to face the heat dissipating part 122. However, the present embodiment is not limited thereto and the inlet pipe 131 may also be disposed at a position where the fluid may be injected into the second space 101b without facing the heat dissipating part 122.

The introduction device 132 may be connected to the inlet pipe 131 and provided to forcibly move the fluid outside the case 101 into the second space 101b of the case 101. The introduction device 132 may include a pump to force the fluid to move. The introduction device 132 may be controlled by the controller 108. Particularly, when a user inputs a command via the input device 20, the controller 108 may control the introduction device 132 such that the introduction device 132 injects the fluid into the case 101 via the inlet pipe 131 upon receiving the command. In this regard, the controller 108 may be provided to control an amount of the fluid supplied by the introduction device 132 as needed. Detailed descriptions thereof will be given later.

The outlet pipe 133 may be provided to discharge the fluid of the second space 101b of the case 101 out of the case 101. That is, the outlet pipe 133 may guide the fluid contained in the second space 101b of the case 101 to the outside of the case 101. In the same manner as the inlet pipe 131, the outlet pipe 133 may be disposed in the cable C. Accordingly, the outlet pipe 133 may penetrate the case 101.

The discharge device 134 is connected to the outlet pipe 133 and provided to forcibly move the fluid inside the second space 101b of the case 101 out of the case 101. The discharge device 134 may include a pump to force the fluid to move. The discharge device 134 may be controlled by the controller 108. Particularly, when the user inputs a command via the input device 20, the controller 108 may control the discharge device 134 such that the discharge device 134 discharges the fluid out of the case 101 via the outlet pipe 133 upon receiving the command. In this regard, the controller 108 may be provided to control an amount of the fluid discharged by the discharge device 134 as needed. Detailed descriptions thereof will be given later.

The aforementioned introduction device 132 and discharge device 134 may be omitted, if required. Particularly, the ultrasonic probe 100 according to the present embodiment may include only the introduction device 132 without having the discharge device 134. In this case, discharging of the fluid contained in the second space 101b of the case 101 may be performed by a pressure difference between the inside and the outside of the case 101. On the contrary, the ultrasonic probe 100 according to the present embodiment may include only the discharge device 134 without having the introduction device 132. In this case, injecting of the fluid into the second space 101b of the case 101 may be performed by a pressure difference between the inside and the outside of the case 101.

Referring to FIG. 4, the fluid cooling apparatus 130 may be provided to adjust the amount of the fluid circulating to the outside of the case 101 in proportion to the amount of heat generated in the driving device 105. That is, when the temperature sensor 105a measures the temperature of the driving device 105 and transmits the measured temperature of the driving device 105 to the controller 108, the controller 108 may adjust the amount of the fluid injected into the second space 101b by the fluid cooling apparatus 130. Particularly, when the temperature of the driving device 105 is relatively high due to a large amount of heat generated in the driving device 105, the controller 108 may control the fluid cooling apparatus 130 to increase an amount of the fluid injected into the second space 101b in which the heat dissipating part 122 is disposed. On the contrary, when the temperature of the driving device 105 is relatively low due to a small amount of heat generated in the driving device 105, the controller 108 may control the fluid cooling apparatus 130 to decrease the amount of the fluid injected into the second space 101b in which the heat dissipating part 122 is disposed.

According to this configuration, the ultrasonic probe 100 according to the present embodiment may quickly cool the heat dissipating part 122 of the thermoelectric element 120.

The partition 140 may divide the inner space of the case 101 into a first space 101a in which the heat absorbing part 121 of the thermoelectric element 120 is disposed and the second space 101b in which the heat dissipating part 122 is disposed. Particularly, the partition 140 may extend from an inner surface of the case 101 to the thermoelectric element 120 to be in contact with the thermoelectric element 120. The partition 140 may contact a boundary of the heat absorbing part 121 and the heat dissipating part 122 of the thermoelectric element 120. The partition 140 may be formed of a heat insulating member such that heat of the second space 101b is not transferred to the first space 101a.

The partition 140 may support edges of the heat absorbing part 121 and the heat dissipating part 122 of the thermoelectric element 120. The partition 140 may be integrally formed with the case 101. Alternatively, the partition 140 may be provided separately from the case 101 and mounted on the case 101.

Since the partition 140 completely divides the inner space of the case 101 into the first space 101a and the second space 101b, transfer of heat generated in the heat dissipating part 122 of the thermoelectric element 120 back to the driving device 105 may be prevented. That is, the ultrasonic probe 100 according to the present embodiment may have a high heat dissipating efficiency by cooling the heat dissipating part 122 by circulating only the fluid contained in the second space 101b in which the heat dissipating part 122 of the thermoelectric element 120 is disposed out of the case 101.

Also, the PCB 109 that is electrically connected to the driving device 105 and controls the ultrasonic probe 100 may be disposed in the first space 101a of the case 101. Since the PCB 109 is disposed in the first space 101a separated from the second space 101b that communicates with the outside, the ultrasonic probe 100 according to the present embodiment may prevent the PCB 109 from being damaged by harmful substances from the outside.

As described above, the ultrasonic probe 100 according to the present embodiment generates a large amount of heat since the transducer array 104 is a 2D transducer array. However, since the thermal conduction member 110 conducts heat generated by the driving device 105 and/or the acoustic module 103 to the thermoelectric element 120, the thermoelectric element 120 cools the thermal conduction member 110, and the fluid cooling apparatus 130 quickly cools the heat dissipating part 122 of the thermoelectric element 120, the ultrasonic probe 100 may have a high heat dissipating efficiency. Thus, the ultrasonic probe 100 according to the present embodiment may not cause inconvenience to the object by preventing heat from being transferred to the object, may not deteriorate the quality of image due to adverse effects of heat on the driving device 105 and/or the PCB 109, and may have high durability by protecting the driving device 105 and/or the PCB 109 from harmful substances of the outside.

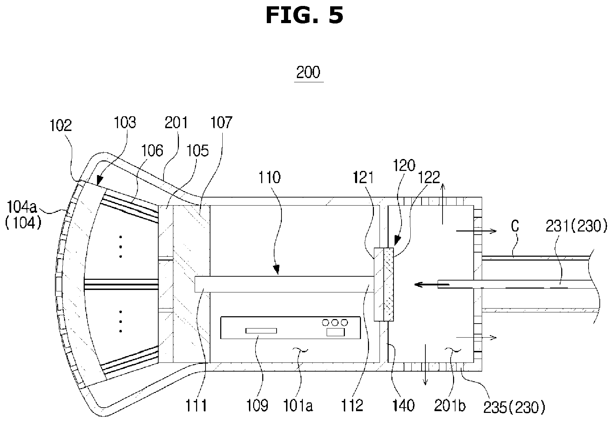

FIG. 5 is a cross-sectional view of an ultrasonic probe 200 according to another embodiment of the present disclosure.

The ultrasonic probe 200 will be described with reference to FIG. 5. The same reference numerals may be applied to the same elements as those illustrated in FIGS. 3 and 4 and descriptions thereof may be omitted.

The ultrasonic probe 200 may include a case 201, an acoustic module 103, a driving device 105, a thermal conduction member 110, a thermoelectric element 120, a fluid cooling apparatus 230, and a partition 140. Here, the acoustic module 103, the driving device 105, the thermal conduction member 110, the thermoelectric element 120, and the partition 140 are the same as those illustrated in FIGS. 3 and 4, and thus detailed descriptions thereof will not be repeated.

The fluid cooling apparatus 230 of the ultrasonic probe 200 may cool the heat dissipating part 122 of the thermoelectric element 120 by circulating a fluid contained in at least one part of the case 201 out of the case 201. The fluid cooling apparatus 230 may include an inlet pipe 231, an introduction device 132, and a discharge hole 235.

The inlet pipe 231 may be provided to move a fluid into the second space 201b of the case 201 from the outside of the case 201. The inlet pipe 231 may be connected to the introduction device 132 to guide the fluid forcibly moved by the introduction device 132 into the second space 201b of the case 201. The inlet pipe 231 may be disposed in the cable C. The introduction device 132 may be controlled by the controller 108 to adjust an amount of the fluid supplied into the second space 201b.

The ultrasonic probe 200 according to the present embodiment may include the discharge hole 235 formed at a portion constituting the second space 201b of the case 201 which is different from the outlet pipe 133 and the discharge device 134 of the ultrasonic probe 100 illustrated in FIGS. 3 and 4.

The discharge hole 235 may be formed to penetrate at least one portion of the case 201 constituting the second space 201b to communicate the second space 201b of the case 201 with the outside of the case 201. The discharge hole 235 may be formed at a left potion and/or a right portion (at an upper portion and/or a lower portion of FIG. 5) of the case 201. Alternatively, the discharge hole 235 may be formed at a rear portion (at a right portion in FIG. 5) of the case 201.

According to this configuration, the fluid cooling apparatus 230 according to the present embodiment may allow the fluid supplied via the inlet pipe 231 to exchange heat with the heat dissipating part 122 of the thermoelectric element 120 and then to be discharged out of the case 201 via the discharge hole 235. That is, the fluid introduced into the second space 201b via the inlet pipe 231 may be discharged out of the case 201 by a pressure difference between the second space 201b of the case 201 and the outside of the case 201 caused by introduction of the fluid into the second space 201b.

According to this configuration, the ultrasonic probe 200 illustrated in FIG. 5 may be manufactured with lower manufacturing cost than the ultrasonic probe 100 illustrated in FIGS. 3 and 4.

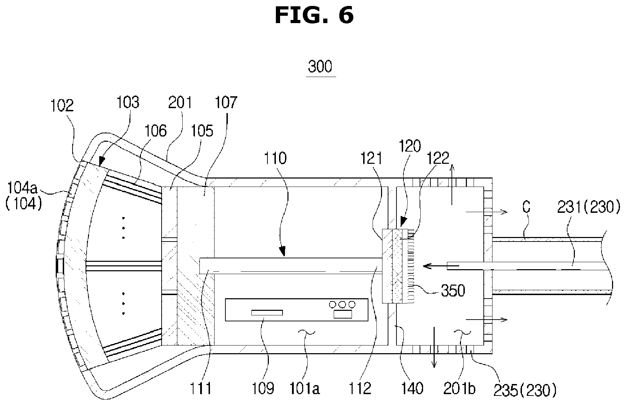

FIG. 6 is a cross-sectional view of an ultrasonic probe 300 according to another embodiment of the present disclosure.

The ultrasonic probe 300 will be described with reference to FIG. 6. The same reference numerals may be applied to the same elements as those illustrated in FIG. 5 and descriptions thereof may be omitted.

The ultrasonic probe 300 may include a case 201, an acoustic module 103, a driving device 105, a thermal conduction member 110, a thermoelectric element 120, a fluid cooling apparatus 230, a partition 140, and a cooling fin 350. Here, the case 201, the acoustic module 103, the driving device 105, the thermal conduction member 110, the thermoelectric element 120, the fluid cooling apparatus 230, and the partition 140 are the same as those illustrated in FIG. 5, and thus detailed descriptions thereof will not be repeated.

The ultrasonic probe 300 may further include the cooling fin 350 to increase a cooling efficiency of the heat dissipating part 122 of the thermoelectric element 120. The cooling fin 350 may be disposed to be adjacent to the heat dissipating part 122.

The cooling fin 350 may contact at least one portion of the heat dissipating part 122 of the thermoelectric element 120. The cooling fin 350 may receive heat from the heat dissipating part 122 of the thermoelectric element 120 and be cooled by the fluid cooling apparatus 230. In this case, since the cooling fin 350 includes a plurality of fins to enlarge a contact area with the fluid supplied by the fluid cooling apparatus 230, the heat dissipating part 122 of the thermoelectric element 120 may be quickly cooled.

The cooling fin 350 may have a size larger than the heat dissipating part 122 of the thermoelectric element 120. Thus, the cooling fin 350 may enlarge a contact area with the fluid supplied by the fluid cooling apparatus 230. The cooling fin 350 may be formed of a material having a high thermal conductivity.

According to this configuration, the ultrasonic probe 300 illustrated in FIG. 6 may have a higher heat dissipating efficiency than that of the ultrasonic probe 200 illustrated in FIG. 5.

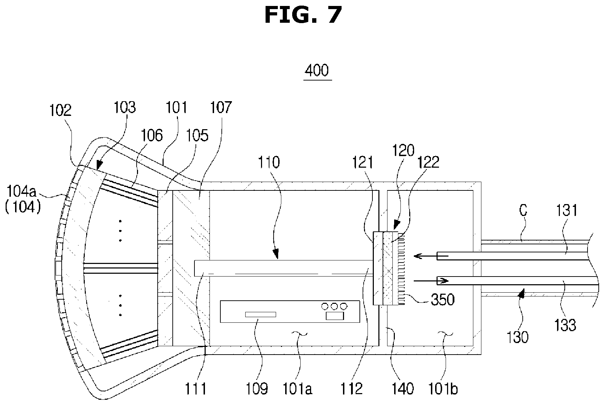

FIG. 7 is a cross-sectional view of an ultrasonic probe 400 according to another embodiment of the present disclosure.

The ultrasonic probe 400 will be described with reference to FIG. 7. The same reference numerals may be applied to the same elements as those illustrated in FIGS. 3 and 4 and descriptions thereof may be omitted.

The ultrasonic probe 400 may include a case 101, an acoustic module 103, a driving device 105, a thermal conduction member 110, a thermoelectric element 120, a fluid cooling apparatus 130, a partition 140, and a cooling fin 350. Here, the case 101, the acoustic module 103, the driving device 105, the thermal conduction member 110, the thermoelectric element 120, the fluid cooling apparatus 130, and the partition 140 are the same as those illustrated in FIGS. 3 and 4, and thus detailed descriptions thereof will not be repeated.

The ultrasonic probe 400 may further include the cooling fin 350 in comparison with the ultrasonic probe 100 illustrated in FIGS. 3 and 4. The configuration of the cooling fin 350 may be the same as that of the cooling fin 350 illustrated in FIG. 6.

That is, the ultrasonic probe 400 according to the embodiment illustrated in FIG. 7 may include the cooling fin 350 in contact with at least one portion of the heat dissipating part 122 of the thermoelectric element 120. Since the cooling fin 350 includes a plurality of fins to enlarge a contact area with the fluid supplied by the fluid cooling apparatus 130, the heat dissipating part 122 of the thermoelectric element 120 may be quickly cooled.

According to this configuration, the ultrasonic probe 400 illustrated in FIG. 7 may have a higher heat dissipating efficiency than the ultrasonic probe 100 illustrated in FIGS. 3 and 4.

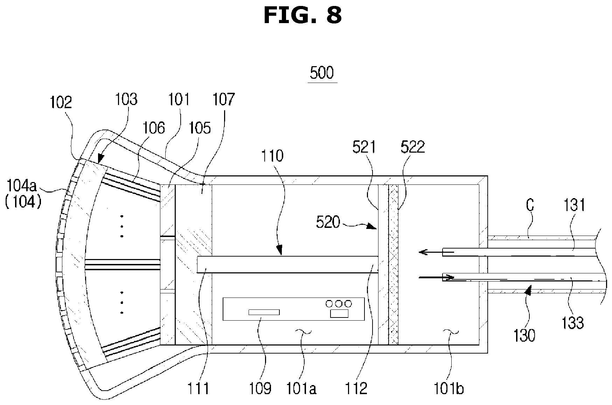

FIG. 8 is a cross-sectional view of an ultrasonic probe 500 according to another embodiment of the present disclosure.

The ultrasonic probe 500 will be described with reference to FIG. 8. The same reference numerals may be applied to the same elements as those illustrated in FIGS. 3 and 4 and descriptions thereof may be omitted.

The ultrasonic probe 500 may include a case 101, an acoustic module 103, a driving device 105, a thermal conduction member 110, a thermoelectric element 520, and a fluid cooling apparatus 130. Here, the case 101, the acoustic module 103, the driving device 105, the thermal conduction member 110, and the fluid cooling apparatus 130 are the same as those illustrated in FIGS. 3 and 4, and thus detailed descriptions thereof will not be repeated.

In the ultrasonic probe 500, a thermoelectric element 520 may divide the inner space of the case 101 into a first space 101a in which a heat absorbing part 521 is disposed and a second space 101b in which a heat dissipating part 522 is disposed, which is different from the embodiment illustrated in FIGS. 3 and 4. Accordingly, the heat absorbing part 521 of the thermoelectric element 520 has a larger area so as to cool the other end 112 of the thermal conduction member 110 more quickly. Since the heat dissipating part 522 also has a larger area, a contact area with a fluid supplied by the fluid cooling apparatus 130 increases so as to cool the heat dissipating part 522 more quickly.

According to this configuration, the ultrasonic probe 500 according to the embodiment illustrated in FIG. 8 may prevent transfer of heat of the second space 101b back to the first space 101a more completely in comparison with the ultrasonic probe 100 illustrated in FIGS. 3 and 4 and may have a higher heat dissipating efficiency by increasing the areas of the heat absorbing part 521 and the heat dissipating part 522.

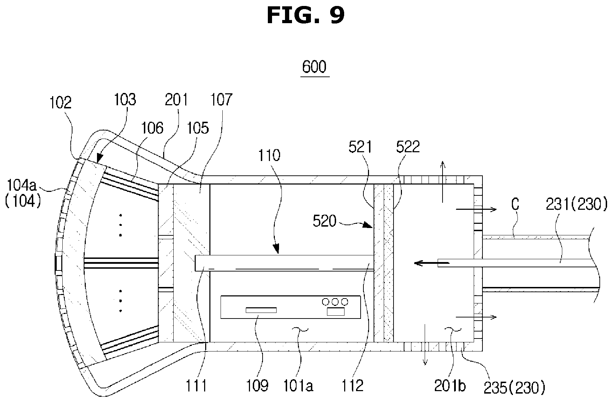

FIG. 9 is a cross-sectional view of an ultrasonic probe 600 according to another embodiment of the present disclosure.

The ultrasonic probe 600 will be described with reference to FIG. 9. The same reference numerals may be applied to the same elements as those illustrated in FIG. 5 and descriptions thereof may be omitted.

The ultrasonic probe 600 may include a case 201, an acoustic module 103, a driving device 105, a thermal conduction member 110, a thermoelectric element 520, and a fluid cooling apparatus 230. Here, the case 201, the acoustic module 103, the driving device 105, the thermal conduction member 110, and the fluid cooling apparatus 230 are the same as those illustrated in FIG. 5, and thus detailed descriptions thereof will not be repeated.

In the ultrasonic probe 600, the thermoelectric element 520 may divide the inner space of the case 101 into the first space 101a in which the heat absorbing part 521 is disposed and the second space 201b in which the heat dissipating part 522 is disposed in the same manner of the ultrasonic probe 500 illustrated in FIG. 8.

According to this configuration, the ultrasonic probe 600 according to the embodiment illustrated in FIG. 9 may prevent transfer of heat of the second space 201b back to the first space 101a via the partition 140 more completely in comparison the ultrasonic probe 200 illustrated in FIG. 5 and may have a higher heat dissipating efficiency by increasing the areas of the heat absorbing part 521 and the heat dissipating part 522.

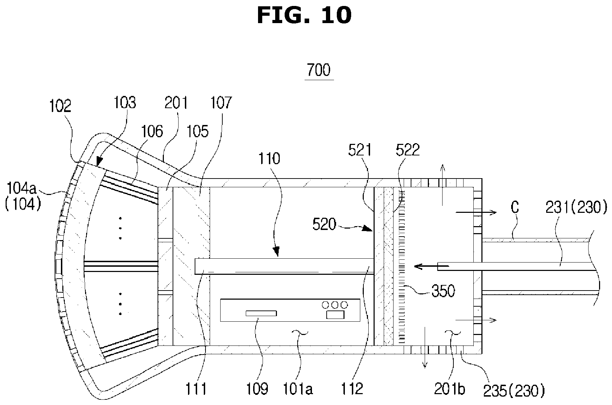

FIG. 10 is a cross-sectional view of an ultrasonic probe 700 according to another embodiment of the present disclosure.

The ultrasonic probe 700 will be described with reference to FIG. 10. The same reference numerals may be applied to the same elements as those illustrated in FIG. 6 and descriptions thereof may be omitted.

The ultrasonic probe 700 may include a case 201, an acoustic module 103, a driving device 105, a thermal conduction member 110, a thermoelectric element 520, and a fluid cooling apparatus 230. Here, the case 201, the acoustic module 103, the driving device 105, the thermal conduction member 110, and the fluid cooling apparatus 230 are the same as those illustrated in FIG. 6, and thus detailed descriptions thereof will not be repeated.

In the ultrasonic probe 700, the thermoelectric element 520 may divide the inner space of the case 101 into the first space 101a in which the heat absorbing part 521 is disposed and the second space 201b in which the heat dissipating part 522 is disposed in the same manner of the ultrasonic probe 500 illustrated in FIG. 8.

According to this configuration, the ultrasonic probe 700 according to the embodiment illustrated in FIG. 10 may prevent transfer of heat of the second space 101b back to the first space 101a via the partition 140 more completely in comparison the ultrasonic probe 200 illustrated in FIG. 6 and may have a higher heat dissipating efficiency by increasing the areas of the heat absorbing part 521 and the heat dissipating part 522.

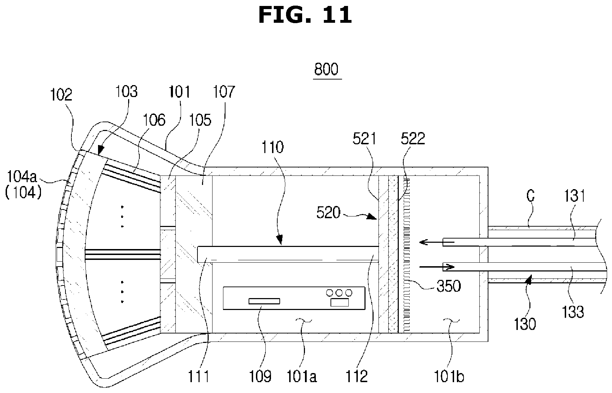

FIG. 11 is a cross-sectional view of an ultrasonic probe 800 according to another embodiment of the present disclosure.

The ultrasonic probe 800 will be described with reference to FIG. 11. The same reference numerals may be applied to the same elements as those illustrated in FIG. 7 and descriptions thereof may be omitted.

The ultrasonic probe 800 may include a case 101, an acoustic module 103, a driving device 105, a thermal conduction member 110, a thermoelectric element 520, and a fluid cooling apparatus 130. Here, the case 101, the acoustic module 103, the driving device 105, the thermal conduction member 110, and the fluid cooling apparatus 130 are the same as those illustrated in FIG. 7, and thus detailed descriptions thereof will not be repeated.

In the ultrasonic probe 800, the thermoelectric element 520 may divide the inner space of the case 101 into the first space 101a in which the heat absorbing part 521 is disposed and the second space 101b in which the heat dissipating part 522 is disposed in the same manner of the ultrasonic probe 500 illustrated in FIG. 8.

According to this configuration, the ultrasonic probe 800 according to the embodiment illustrated in FIG. 11 may prevent transfer of heat of the second space 101b back to the first space 101a via the partition 140 more completely in comparison the ultrasonic probe 400 illustrated in FIG. 7 and may have a higher heat dissipating efficiency by increasing the areas of the heat absorbing part 521 and the heat dissipating part 522.

As is apparent from the above description, an ultrasonic probe according to the present disclosure may have a high heat dissipating efficiency by forcibly circulating a fluid.

According to the present disclosure, the ultrasonic probe may have a high heat dissipating efficiency by preventing transfer of heat back to the driving device by separating the heat absorbing part of the thermoelectric element from a heat dissipating part thereof.

According to the present disclosure, the ultrasonic probe may have a high image quality by increasing heat dissipating effects.

According to the present disclosure, the ultrasonic probe may protect the PCB from external substances by dividing the inner space of the case into a space in which the PCB is disposed and a space communicating with the outside.

Although a few embodiments of the present disclosure have been shown and described, it would be appreciated by those skilled in the art that changes may be made in these embodiments without departing from the principles and spirit of the disclosure, the scope of which is defined in the claims and their equivalents.

## Claims

1. An ultrasonic probe comprising:
a case;
a transducer array disposed in the case and configured to generate ultrasound;
a driving device disposed in the case and configured to drive the transducer array;
a thermal conduction member connected to the driving device and having thermal conductivity;
a thermoelectric element comprising a heat absorbing part and a heat dissipating part, the heat absorbing part disposed to face the thermal conduction member to cool the thermal conduction member;
a fluid cooling apparatus configured to cool the heat dissipating part of the thermoelectric element by circulating a fluid of at least one portion of the inside of the case to the outside of the case; and
a partition configured to divide an inner space of the case into a first space in which the heat absorbing part of the thermoelectric element is disposed and a second space in which the heat dissipating part is disposed.

2. The ultrasonic probe according to claim 1, wherein the partition extends from an inner surface of the case toward the thermoelectric element to be in contact with the thermoelectric element.

3. The ultrasonic probe according to claim 2, wherein the partition is in contact with a boundary of the heat absorbing part and the heat dissipating part of the thermoelectric element.

4. The ultrasonic probe according to claim 1, wherein the fluid cooling apparatus is provided to adjust an amount of the fluid circulating to the outside of the case in proportion to an amount of heat generated by the driving device.

5. The ultrasonic probe according to claim 1, wherein the thermoelectric element adjusts a temperature difference between the heat absorbing part and the heat dissipating part in proportion to an amount of heat generated by the driving device.

6. The ultrasonic probe according to claim 1, wherein the fluid cooling apparatus comprises:
an inlet pipe configured to move a fluid outside the case into the second space of the case; and
an introduction device connected to the inlet pipe and configured to forcibly move the fluid outside the case into the second space of the case.

7. The ultrasonic probe according to claim 6, further comprising a cable penetrating one side of the case to supply power to the driving device,
wherein the inlet pipe is disposed inside the cable.

8. The ultrasonic probe according to claim 1, wherein the fluid cooling apparatus comprises an outlet pipe configured to discharge the fluid of the second space of the case out of the case.

9. The ultrasonic probe according to claim 8, wherein the fluid cooling apparatus comprises a discharge device connected to the outlet pipe and configured to forcibly move the fluid of the second space of the case out of the case.

10. The ultrasonic probe according to claim 1, wherein the fluid cooling apparatus comprises a discharge hole formed at one portion of the case constituting the second space of the case and penetrating the case to discharge the fluid of the second space of the case out of the case.

11. The ultrasonic probe according to claim 1, further comprising a cooling fin disposed adjacent to the heat dissipating part of the thermoelectric element.

12. The ultrasonic probe according to claim 1, further comprising a printed circuit board (PCB) electrically connected to the driving device,
wherein the PCB is disposed in the first space of the case.

13. The ultrasonic probe according to claim 1, further comprising a thermal conduction block having a size corresponding to the driving device in contact with one surface of the driving device facing the thermal conduction member.

14. The ultrasonic probe according to claim 1, wherein the transducer array comprises transducer elements arranged two-dimensionally.

15. The ultrasonic probe according to claim 1, wherein the thermal conduction member comprises a heat pipe.
